## Europäisches Patentamt
## European Patent Office
## Office européen des brevets

(19)

(11) Veröffentlichungsnummer : **0 118 724**
**B1**

(12)

# EUROPÄISCHE PATENTSCHRIFT

(45) Veröffentlichungstag der Patentschrift :
22.10.86

(51) Int. Cl.⁴ : **A 61 B 5/14**

(21) Anmeldenummer : **84101033.3**

(22) Anmeldetag : **02.02.84**

(54) **Verschlusskappe für ein Röhrchen für die Blutentnahme.**

(30) Priorität : **12.02.83 DE 8304179 U**

(43) Veröffentlichungstag der Anmeldung :
**19.09.84 Patentblatt 84/38**

(45) Bekanntmachung des Hinweises auf die Patenterteilung : **22.10.86 Patentblatt 86/43**

(84) Benannte Vertragsstaaten :
**AT BE CH DE FR GB IT LI LU NL SE**

(56) Entgegenhaltungen :
**CH-B-   476 501**
**DE-A- 2 415 496**
**DE-A- 2 455 631**
**DE-A- 2 711 336**
**DE-A- 3 018 262**
**GB-A- 1 327 024**
**US-A- 2 701 566**
**US-A- 3 159 159**
**US-A- 3 508 653**
**US-A- 4 008 718**
**US-A- 4 175 559**

(73) Patentinhaber : **Ballies, Uwe Werner, Dr.**
**Jägersberg 7 - 9**
**D-2300 Kiel (DE)**

(72) Erfinder : **Ballies, Uwe Werner, Dr.**
**Jägersberg 7 - 9**
**D-2300 Kiel (DE)**

(74) Vertreter : **UEXKÜLL & STOLBERG Patentanwälte**
**Beselerstrasse 4**
**D-2000 Hamburg 52 (DE)**

## Beschreibung

Die Erfindung betrifft eine Verschlußkappe für ein Röhrchen insb. für die Blutentnahme, mit einem Boden, mit einem auf dem Boden angeordneten Kanülenkonus und mit einer an den Boden anschließenden Schürze, die eine Verriegelungseinrichtung zur Befestigung der Verschlußkappe auf dem Zylinderkörper des Röhrchens aufweist.

Es ist bereits bekannt, Injektionsspritzen mit einer abnehmbaren Verschlußkappe zu versehen, um beispielsweise eine für den Mehrfachgebrauch bestimmte Spritze besser reinigen zu können. Außerdem kann durch eine abnehmbare Verschlußkappe eine Anpassung von Kanülen unterschiedlichen Durchmessers an ein und denselben Injektionsspritzenzylinder vorgenommen werden. Die Verriegelung der Verschlußkappe gegenüber dem Zylinderkörper der Injektionsspritze erfolgt üblicherweise durch ein Gewinde, und zwar in der Weise, daß die Schürze der Verschlußkappe mit einem Innengewinde und das untere Ende des Injektionsspritzenzylinders mit einem Außengewinde versehen ist. Als Verriegelungseinrichtung sind aber auch schon Bajonettverschlüsse sowie Schnappverschlüsse bekannt.

Wird eine derartige Injektionsspritze zur Blutentnahme verwendet, so sticht der Arzt oder die Krankenschwester die Kanüle mit zunächst vollständig in Richtung Verschlußkappe gedrücktem Stempel in die Vene eines Patienten und zieht dann den mit dem Spritzenkolben verbundenen Stempel hoch. Durch dieses Hoch- oder Aufziehen wird das Blut in den Spritzenzylinder gesaugt. Dies erfordert zwei Hände, denn mit einer Hand muß die in die Vene gestochene Spritze festgehalten werden, während mit der anderen Hand das Aufziehen des Kolbens durchgeführt werden muß.

Es ist Aufgabe der Erfindung, eine verbesserte Verschlußkappe für ein Röhrchen für die Blutentnahme o. ä. zu schaffen, die einen Einhandbetrieb bei der Entnahme von einer Flüssigkeit aus einem Gefäß, insbesondere bei der Entnahme von Blut aus einer Vene, gestattet.

Zur Lösung dieser Aufgabe dient eine Verschlußkappe der eingangs erwähnten Art, welche dadurch gekennzeichnet ist, daß in die Schürze der Verschlußkappe ein Absperrkörper eingesetzt ist, der in unbetätigter Stellung mit einer Absperrfläche eine zu dem Kanülenkonus führende Kanülenbohrung absperrt und der durch Niederdrücken eines Betätigungselements die Kanülenbohrung freigibt. Das Betätigungselement ist vorzugsweise ein Druckknopf, eine Drucktaste, ein Hebel o. ä.

Dadurch wird erreicht, daß der Kolben eines mit der erfindungsgemäßen Verschlußkappe versehenen Röhrchens o. ä., etwa eines Röhrchens gemäß DE-OS 27 11 336.6, bereits vor dem Einstechen in ein Blutgefäß eines Patienten oder in ein irgendeine Behandlungsflüssigkeit enthaltendes Gefäß, beispielsweise eine Flasche, ein Becher o. ä. aufziehbar und in aufgezogener Stellung verriegelbar ist. Durch den Absperrkörper wird dabei die Kanülenbohrung geschlossen gehalten, so daß im Zylinderkörper des Röhrchens o. ä. ein Unterdruck entsteht, der bei Niederdrücken eines auf den Absperrkörper wirkenden Druckknopfs belüftet wird. Wenn zum Zeitpunkt des Niederdrückens des Absperrkörpers die auf die Verschlußkappe aufgesetzte Kanüle in Blut oder in eine andere Flüssigkeit taucht, wird dieses oder diese von dem Unterdruck im Röhrchen in dieses gesaugt, und zwar kann dieses Ansaugen einhändig durchgeführt werden, denn zum Niederdrücken des Druckknopfs reicht ein Finger der die Spritze haltenden Hand aus.

Weitere vorteilhafte Ausgestaltungen der Erfindung ergeben sich aus den Unteransprüchen und der nachfolgenden Figurenbeschreibung.

Die Erfindung wird im folgenden anhand von einer Zeichnung näher erläutert ; es zeigen :

Figur 1 eine Explosionszeichnung eines ersten Ausführungsbeispiels ;

Figur 2 einen Vertikalschnitt durch das zusammengesetzte erste Ausführungsbeispiel ;

Figur 3 einen Vertikalschnitt ähnlich wie Figur 2, jedoch durch ein zweites Ausführungsbeispiel ; und

Figur 4 einen Vertikalschnitt ähnlich wie Figur 3, jedoch durch ein drittes Ausführungsbeispiel.

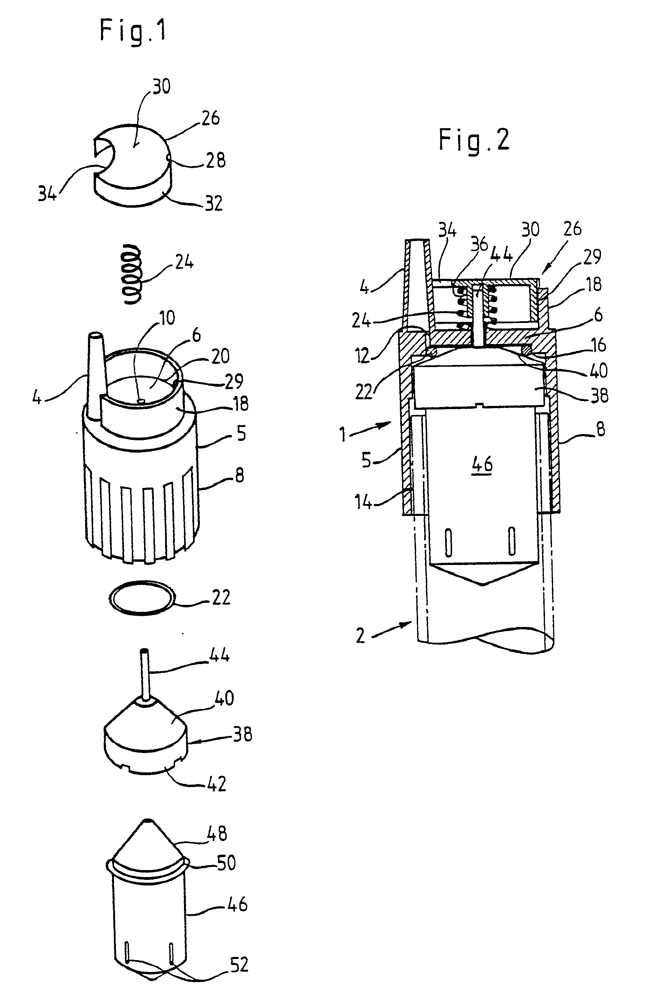

In den Figuren sind gleiche Teile durchweg mit gleichen Bezugszeichen versehen. Figur 1 ist eine Explosionszeichnung einer Verschlußkappe 1, die auf das untere Ende eines in Figur 2 lediglich strichpunktiert angedeuteten Röhrchens 2 für die Blutentnahme o. ä. aufgesetzt wird. Die Verschlußkappe 1 weist gemäß Figur 1 sechs Einzelteile auf, und zwar von oben nach unten einen Druckknopf 26, eine Druckfeder 24, einen Kappenkörper 5, einen O-Ring 22, einen Absperrkörper 38 und ein Trennelement 46. Bis auf die Druckfeder 24 und den O-Ring 22 sind alle anderen Teile im Spritzgußverfahren ohne Schwierigkeiten herstellbar.

Das in den Figuren 1 bis 3 dargestellte Trennelement 46 ist ein zylindrischer Vollkörper, der an seinem unteren Rand Führungsvorsprünge 52 aufweist. Der Außendurchmesser des Trennelements 46 ist so zu wählen, daß es leicht im Inneren eines Röhrchens 2 für die Blutentnahme o. ä. verschiebbar ist, worin es durch die Führungsvorsprünge 52 geführt wird. In einer Ausführung sind vier in axialer Richtung des Trennelements 46 ausgerichtete, längliche Führungsvorsprünge 52 vorgesehen. An seinem den Führungsvorsprüngen 52 gegenüberliegenden, oberen Ende weist das Trennelement 46 einen Oberrand 50 zur Aufnahme des Absperrkörpers 38 auf. Oberhalb des Oberrandes 50 schließt das Trennelement 46 durch eine kegelförmige Zylinderspitze 48 ab. Das Trennelement 46 ist für die Funktionsweise der erfindungsgemäßen Verschlußkappe nicht unbedingt erforderlich.

Auf die kegelförmige Zylinderspitze 48 des Trennelements 46 ist der Absperrkörper 38 aufsetzbar, der eine ebenfalls kegelförmige Absperrfläche 40 und einen sich an die Basis der kegelförmigen Absperrfläche 40 anschließenden Zylinderabschnitt 42 aufweist. Der Innendurchmesser des Zylinderabschnitts 42 entspricht etwa dem Außendurchmesser des sich in Form eines Umfangsflansches um das Trennelement 46 erstreckenden Oberrandes 50, so daß der Zylinderabschnitt 42 zur Verriegelung gegenüber dem Trennelement 46 über den Oberrand 50 schnappt. Der Kegelwinkel der Absperrfläche 40 entspricht in etwa dem Kegelwinkel der Zylinderspitze 48, so daß der über den Oberrand 50 geschnappte Absperrkörper 38 von der Zylinderspitze 48 unterstützt wird. An das obere Ende der Absperrfläche 40 ist eine Spitze 44 angeformt, welche die Form einer Zylinderstange hat.

Der Kappenkörper 5 weist einen Boden 6 auf, welcher einen exzentrisch angeordneten Kanülenkonus 4 trägt. An seiner dem Kanülenkonus 4 abgewandten Seite schließt eine Schürze 8 an, die an ihrer Innenseite mit einem Innengewinde 14 zur Befestigung auf dem Röhrchen 2 versehen ist. Der Kanülenkonus 4 besitzt eine Kanülenbohrung 12, die sich durch den Boden 6 erstreckt und damit das Innere des Kappenkörpers 5 mit dem Kanülenkonus 4 in Strömungsverbindung bringt. Die Kanülenbohrung 12 mündet in der Nähe einer Schulter 16 im Boden 6, und zwar derart, daß ein auf die Innenseite des Bodens 6 gelegter und von der Schulter 16 abgestützter O-Ring 22 die Kanülenbohrung 12 abzudecken vermag.

Auf der dem Kanülenkonus 4 zugewandten Seite des Bodens 6 umgibt eine ringförmige Wand 18 eine Zentralbohrung 10. Der Durchmesser der Zentralbohrung 10 ist geringfügig größer als der Außendurchmesser der Spitze 44 des Absperrkörpers 38, wobei das Übermaß von dem Fachmann ohne Schwierigkeiten wählbar ist. Wenige zehntel Millimeter reichen im allgemeinen aus, um eine freie Verschiebbarkeit der Spitze 44 in der Zentralbohrung 10 zuzulassen. Die Höhe der Wand 18, und zwar in axialer Erstreckung der Verschlußkappe 1 gemessen, ist üblicherweise so gewählt, daß damit die Führung des Druckknopfs 26 möglich ist.

Der Druckknopf 26 ist eine im wesentlichen zylindrische Kappe mit einem bis auf einen Ausschnitt 34 geschlossenen Deckel 30, an den eine zylindrische Seitenwand 32 anschließt. Der Ausschnitt 34 ist an der Stelle des Kanülenkonus 4 vorgesehen und ermöglicht daher trotz des exzentrisch angesetzten Kanülenkonus 4 ein Niederdrücken des Druckknopfs 26. Dem Ausschnitt 34 liegt diametral gegenüber eine Rinne 28, die in Richtung einer Erzeugenden der Seitenwand 32 verläuft, so daß die Rinne 28 über die ganze Höhe der Seitenwand 32 eingeformt ist. Bei montiertem Druckknopf 26 greift ein an der Innenseite der Wand 18 vorgesehener axialer Vorsprung 29 in die Rinne 28 und sichert den Druckknopf 26 gegen radiales Verdrehen, während eine axiale Verschiebung weiterhin möglich ist. Ferner ist an der Unterseite des Deckels 30 des Druckknopfs 26 ein Stiftaufnehmer 36 in Form eines Hohlzylinders angeformt, dessen Höhe etwa so groß wie die Höhe der Seitenwand 32 ist. Der Innendurchmesser des Stiftaufnehmers 36 ist so bemessen, daß er im Preßsitz auf die Spitze 44 des Absperrkörpers 38 aufbringbar ist. Dadurch wird eine kraftschlüssige Verbindung zwischen dem Absperrkörper 38 und dem Druckknopf 26 hergestellt.

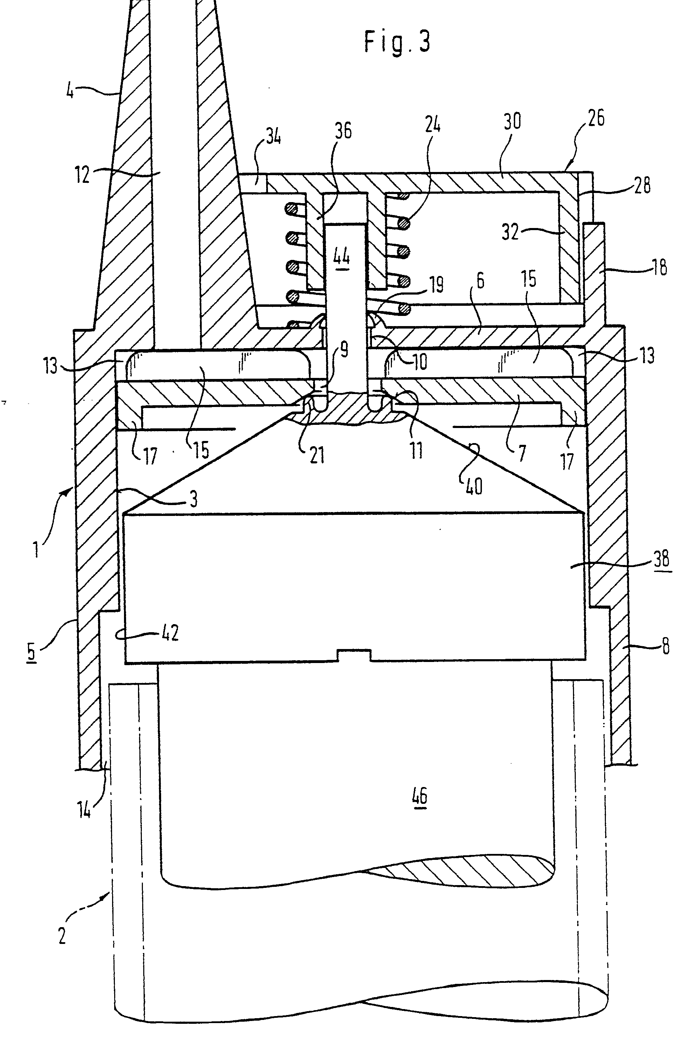

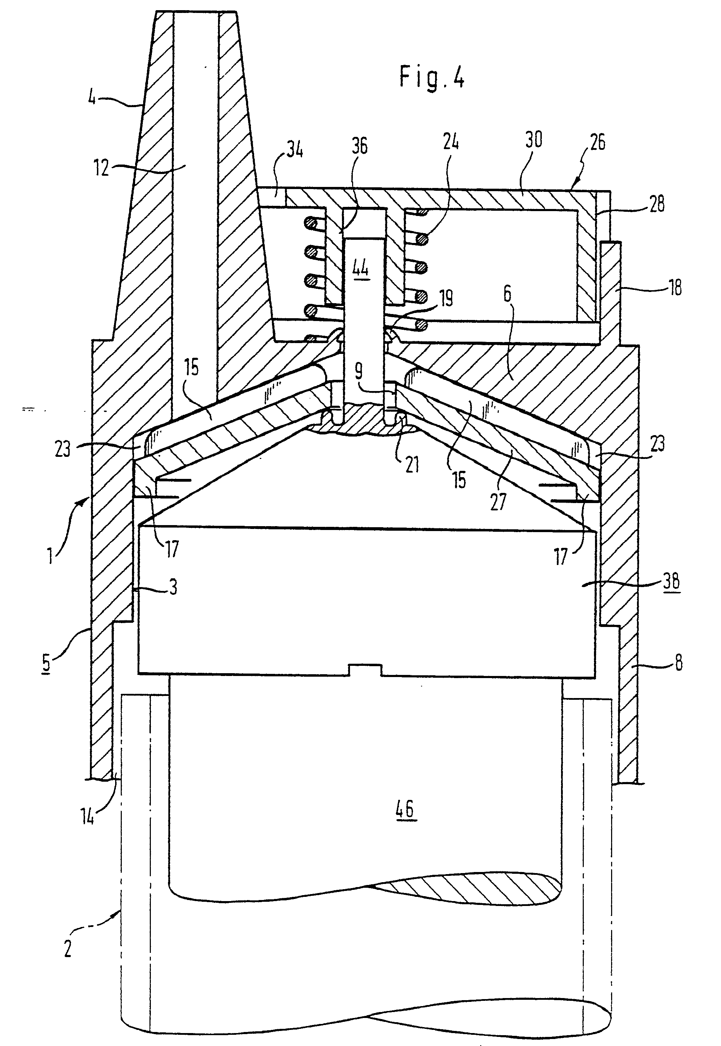

Zwischen den Deckel 30 des Druckknopfs 26 und den Boden 6 des Kappenkörpers 5 ist eine den Stiftaufnehmer 36 umgebende Druckfeder 24 eingesetzt, welche den Druckknopf 26 normalerweise vom Boden 6 weg drückt und damit den Absperrkörper 38 gegen die Unterseite des Bodens 6 zieht. Durch geeignete Wahl des Kegelwinkels der Absperrfläche 40 ist es daher möglich, den O-Ring 22 mit der Absperrfläche 40 abzudichten und gegen den Boden 6 zu pressen. Dabei wird der O-Ring 22 auf die Kanülenbohrung 12 gedrückt, so daß der Kanülenkonus 4 abgesperrt ist. Außerdem erfolgt eine Abdichtung der Zentralbohrung 10, da keine zwischen O-Ring 22 und Absperrfläche 40 vorhandene Luft in den von der Schürze 8 umgebenen Raum gelangen kann. Es ist klar, daß die Kanülenbohrung 12 nicht unbedingt an der Auflagestelle des O-Rings 22 zu liegen braucht; sie muß lediglich innerhalb des O-Ringes 22 angeordnet sein. Ferner läßt sich gegebenenfalls auch ohne O-Ring durch entsprechende Ausbildung der Anlageflächen für die Absperrfläche 40 eine solche Abdichtung erzielen, wie dies bei der Ausführung gemäß Figur 3 oder 4 der Fall ist.

Die Montage der erfindungsgemäßen Verschlußkappe 1 erfolgt in der Weise, daß zunächst der Absperrkörper 38 auf das Trennelement 46 gedrückt wird. Anschließend wird der O-Ring 22 in die von der Schulter 16 im Boden 6 gebildete Vertiefung gelegt. Hierauf wird die Spitze 44 durch die Zentralbohrung 10 des Bodens 6 gesteckt, so daß sie innerhalb der Wand 18 aus dem Boden 6 herausragt. Dann wird die Druckfeder 24 über die Spitze 44 gesetzt und anschließend der Druckknopf 26 auf die Spitze 44 gedrückt, und zwar so weit, daß bei vollständig auf dem O-Ring 22 aufliegender Absperrfläche 40 noch ein Niederdrücken des Druckknopfes 26 möglich ist. Ein Dichtungskragen 19 dichtet die Spitze 44 gegenüber der Zentralbohrung 10 ab.

Die derart montierte Verschlußkappe 1 läßt sich nun ohne Schwierigkeiten auf das Röhrchen 2 für die Blutentnahme o. ä. schrauben, stecken oder auf andere, bekannte Weise aufbringen. Zum Betrieb wird ein an sich bekannter und in den Figuren nicht dargestellter Kolben mit seinem Stempel in das der Verschlußkappe 1 abgewandte Ende des Röhrchens 2 eingebracht und mit einer weiteren, ebenfalls bekannten Kappe gesichert, die zweckmäßigerweise eine Verriegelungseinrichtung aufweist, um den aufgezogenen Kolben in aufgezogener Stellung festzuhalten. Dies wird in an sich bekannter Weise dadurch erreicht, daß auf dem Stempel oder der Kolbenstange Verdickungen vorgesehen sind, die bei kräftiger Zuganwendung durch die Kappe bewegt werden können und ein Zurückrutschen des Kolbens verhindern. Wird dieses Aufziehen und Verriegeln des Kolbens bei unbetätigtem Druckknopf 26 vorgenommen, so entsteht dabei im Röhrchen 2 ein Unterdruck, der dieses betriebsbereit macht. Zum Einstechen in ein Blutgefäß o. ä. wird nun noch eine Kanüle auf den Kanülenkonus 4 gesetzt und die derart montierte Spritze kann einhändig in das Gefäß gestochen werden. Nach dem Einstechen wird der Druckknopf 26 mit einem Finger niedergedrückt, und zwar beispielsweise mit dem Zeigefinger, wodurch das in der nicht dargestellten Kanüle sowie in dem Kanülenkonus 4 stehende Blut oder die Flüssigkeit von dem Unterdruck im Röhrchen 2 schnell durch die Kanülenbohrung 12 in das Röhrchen 2 gesaugt wird.

Nach Aufhebung des Vakuums verschließt der O-Ring 22 wieder die Kanülenbohrung 12 und verhindert den Austritt von Blut aus dieser. Durch Versiegelung des Druckknopfes 26 kann eine Sicherung gegen die Entnahme von But erfolgen.

Soll das im Zylinder enthaltene Blut einer Zentrifugierung unterworfen werden, so kann das Trennelement 46 bis zu einer vorgegebenen g-Zahl mittels seines Oberrandes 50 in verriegeltem Eingriff mit dem Absperrkörper 38 und damit in der Lage gemäß Figur 2 gehalten werden, während eine Vortrennung der Blutbestandteile stattfindet. Erst beim Überschreiten der vorgegebenen g-Zahl erfolgt ein Lösen des Trennelements 46 vom Absperrkörper 38, wie dies beispielsweise in der DE-OS 27 11 336 beschrieben ist.

Figur 3 zeigt ein zweites Ausführungsbeispiel, bei dem die Kanülenbohrung 12 nicht durch einen O-Ring, sondern durch einen in einer ebenen Zwischenwand 7 vorgesehenen Ventilsitz 11 absperrbar ist. Die Zwischenwand 7 trägt auf einer Seite, die im montierten Zustand dem Boden 6 zugewandt ist, eine Anzahl von Rippen 15, in einer Ausführungsform beispielsweise sechs Rippen, die sternförmig um eine Mittelbohrung 9 der Zwischenwand 7 angeordnet sind und in bezug auf diese radial verlaufen. Die Rippen 15 enden zweckmäßigerweise im Abstand sowohl zum Rand 17 der Zwischenwand 7 als auch zum Rand der Mittelbohrung 9, um eine möglichst ungehinderte Verteilung des durch die Kanülenbohrung 12 angesaugten Blutes in einem zwischen dem Boden 6 und der Zwischenwand 7 gebildeten Strömungskanal 13 zu gestatten. Die Mittelbohrung 9 hat einen so großen Durchmesser, daß sich die Spitze 44 des Absperrkörpers 38 ungehindert hindurchbewegen kann und außerdem ein freier Blutströmungsweg durch den zwischen der Spitze 44 und der Wand der Mittelbohrung 9 gebildeten Ringraum besteht. Zweckmäßigerweise ist der Durchmesser der Mittelbohrung 9 1,2 bis 2 mal so groß wie der Außendurchmesser der Spitze 44. Die Mittelbohrung 9 ist außerdem koaxial zur Zentralbohrung 10 angeordnet, so daß sich die Spitze 44 des Absperrkörpers 38 ohne zu verkanten oder zu klemmen gleichzeitig durch die Mittelbohrung 9 und durch die Zentralbohrung 10 erstrecken kann. Die Zentralbohrung 10 hat einen kleineren Durchmesser als die Mittelbohrung 9, jedoch einen geringfügig größeren Durchmesser als die Spitze 44, so daß diese unter leichter Reibung in der Zentralbohrung 10 in axialer Richtung verschiebbar ist. Zur Abdichtung der Spitze 44 ist auf der Außenseite des Bodens 6, welche dem Druckknopf 26 zugewandt ist, wiederum ein Dichtungskragen 19 vorgesehen, der den Austritt von Blut aus dem Raum zwischen der Zentralbohrung 10 und der Spitze 44 verhindert. Hierzu berührt der obere Rand des Dichtungskragens 19 den Umfang der Spitze 44, die zu diesem Zweck vorteilhafterweise einen zylindrischen Querschnitt hat.

Der Rand 17 der ebenen Zwischenwand 7 ist in der dargestellten Ausführungsform etwas nach unten in Richtung auf das Röhrchen 2 verlängert, um ein besseres Anliegen an der Innenwand 3 der Verschlußkappe 1 zu gestatten und ein Verkanten der Zwischenwand 7 beim Einsetzen in die Verschlußkappe 1 zu verhindern. An die Mittelbohrung 9 schließt ein Ventilsitz 11 an, der sich nach außen und unten, also in Richtung von den Rippen 15 weg, kegelförmig erweitert und eine Dichtungsfläche für einen Ringkragen 21 bildet, der den Fuß der Spitze 44 umgibt und damit das spitzenseitige Ende der Absperrfläche 40 bildet. Bei dem in Figur 3 dargestellten Ausführungsbeispiel erfolgt somit das Öffnen oder Schließen des Ventils nicht durch die Absperrfläche 40, sondern durch den Ringkragen 21, der durch die Wirkung der Druckfeder 24 über den Stiftaufnehmer 36 und über die von dem Stiftaufnehmer 36 mit Preßsitz gehaltene Spitze 44 auf den Ventilsitz 11 gezogen wird. Das Öffnen des Ventils erfolgt durch einfaches Niederdrücken des Druckknopfs 26, wodurch der Ringkragen 21 vom Ventilsitz 11 abhebt und Blut durch die Kanülenbohrung 12, durch den Strömungskanal 13 und durch die Mittelbohrung 9 in das Röhrchen 2 einströmen kann.

Figur 4 zeigt ein drittes Ausführungsbeispiel der Erfindung, das weitgehend dem Ausführungsbeispiel gemäß Figur 3 entspricht. Gleiche Teile sind daher wiederum mit gleichen Bezugszeichen versehen. Das Ausführungsbeispiel gemäß Figur 4 unterscheidet sich jedoch von der Ausführungsform nach Figur 3 in der Gestaltung der Zwischenwand 27, die keine ebene Platte, sondern ein kegelförmiger Hut ist. Die Zwischenwand 27 ist an ihrer Oberseite wiederum mit radial verlaufenden Rippen 15 versehen, welche einen Abstand zwischen dem Boden 6 und der Zwischenwand 27 herstellen und damit einen kegelförmigen Strömungskanal 23 einrichten. Der Rand 17 der Zwischenwand 27 ist wiederum zum besseren Anliegen an der Innenwand 3 der Verschlußkappe 1 in axialer Richtung nach unten verlängert. In der Mitte der Zwischenwand 27 ist die Mittelbohrung 9 für den Durchtritt der Spitze 44 des Absperrkörpers 38 vorgesehen, welche mit der Spitze 44 den anhand von Figur 3 erläuterten Ringraum für den Durchtritt von Blut bildet. Zum Abdichten des Strömungskanals 23 gegenüber dem Inneren des Röhrchens 2 dient wiederum der am Fuß der Spitze 44 vorgesehene Ringkragen 21, der sich in dieser Ausführungsform jedoch direkt gegen die Unterseite der Zwischenwand 27 legt. Die Unterseite der Zwischenwand 27 bildet somit einen Ventilsitz. Es ist dem Fachmann klar, daß gegebenenfalls ein zusätzlicher Ventilsitz vorgesehen sein sollte, falls die Neigung der Unterseite der Zwischenwand 27 keine Auflage bildet, die den Strömungskanal 23 zuverlässig absperrt. Mit Ausnahme der Druckfeder 24 sind alle Teile der in den Figuren 1 bis 4 dargestellten Verschlußkappe 1 zweckmäßigerweise aus Kunststoff im Spritzgußverfahren hergestellt. Ferner ist in einer nicht dargestellten Ausführung das Trennelement 46 nicht in den Absperrkörper 38 eingeschnappt, sondern über eine Sollbruchstelle mit diesem verbunden, die bei Erreichen einer bestimmten g-Zahl während des Zentrifugierens aufbricht und das Trennelement 46 freigibt. Beim Zentrifugieren erfolgt daher zunächst eine Vorseparierung ohne Trennelement 46, das sich erst bei Erhöhung auf eine bestimmte Geschwindigkeit vom Absperrkörper 38 löst.

Die erfindungsgemäße Verschlußkappe hat den Vorteil, daß sie in Verbindung mit Normalkanülen verwendbar ist, die üblicherweise einen Luerkonus aufweisen. Durch die exzentrische Anordnung des Kanülenkonus 4 und durch den Druckknopf 26 ist ein Einhandbetrieb möglich, wobei die Verschlußkappe 1 mit dem eingeschraubten oder auf sonstige Weise mit ihr verriegelten Röhrchen 2 zum Punktieren eines Blutgefäßes zwischen Daumen und Mittelfinger gehalten wird, während der Zeigefinger auf dem Druckknopf 26 ruht. Ob ein Blutgefäß beim Einstechen tatsächlich getroffen wurde, läßt sich durch kurzfristiges Niederdrücken des Druckknopfs 26 ohne weiteres feststellen, was beim Stand der Technik bislang nicht möglich war. Außerdem kann das von dem Röhrchen 2 zum Ansaugen von Blut dienende Vakuum immer wieder durch Schließen des Ventils in der Verschlußkappe 1 unterbrochen werden, falls ein Kollabieren einer Vene befürchtet wird.

Es wird darauf hingewiesen, daß anstelle des Druckknopfs auch anders gebaute Betätigungselemente zum Einhandbetrieb der Verschlußkappe geeignet sind, beispielsweise Drucktasten, Hebel oder andere Einrichtungen, die entweder durch Kunststoff-Federelemente in ihre Ausgangsstellung zurückgestellt werden können oder die ohne jegliches Federelement durch Betätigung von einem oder mehreren Hebeln in ihre Ausgangsstellung zurückführbar sind.

Es wird ferner darauf hingewiesen, daß das Vakuum in dem Röhrchen nicht unbedingt durch Aufziehen des Kolbens geschaffen werden muß, sondern bereits vor dem Gebrauch vorhanden sein kann, indem beispielsweise bereits bei der Herstellung das Röhrchen evakuiert wird. Dies kann auf verschiedenste Weise erfolgen, beispielsweise durch Montage in der Vakuumkammer, durch Vorsehen eines Rückschlagventils, über welches evakuiert werden kann, oder auf andere, dem Fachmann geläufige Weise.

**Patentansprüche**

1. Verschlußkappe für ein Röhrchen insb. für die Blutentnahme,
— mit einem Boden (6) ;
— mit einem auf dem Boden (6) angeordneten Kanülenkonus (4) ; und
— mit einer an den Boden (6) anschließenden Schürze (8), die eine Verriegelungseinrichtung zur Befestigung der Verschlußkappe (1) auf dem Röhrchen (2) aufweist,
dadurch gekennzeichnet,
— daß in die Schürze (8) der Verschlußkappe (1) ein Absperrkörper (38) eingesetzt ist, der in unbetätigter Stellung eine zu dem Kanülenkonus (4) führende Kanülenbohrung (12) absperrt und der durch Niederdrücken eines Betätigungselements (26) die Kanülenbohrung (12) freigibt.

2. Verschlußkappe nach Anspruch 1, dadurch gekennzeichnet, daß der Absperrkörper (38) eine Spitze (44) aufweist, die durch den Boden (6) der Verschlußkappe (1) ragt und mit dem Betätigungselement (26) in Eingriff steht.

3. Verschlußkappe nach Anspruch 2, dadurch gekennzeichnet, daß das Betätigungselement (26) ein Druckknopf, eine Drucktaste, ein Hebel o. ä. ist.

4. Verschlußkappe nach Anspruch 3, dadurch gekennzeichnet, daß die Spitze (44) des Absperrkörpers (38) in einen unter dem Deckel (30) des Druckknopfs (26) angeordneten Stiftaufnehmer (36) formschlüssig oder kraftschlüssig eingreift, und daß der Stiftaufnehmer (36) von einer sich zwischen dem Deckel (30) und dem Boden (6) abstützenden Druckfeder (24) umgeben ist.

5. Verschlußkappe nach einem der Ansprüche 1 bis 4, dadurch gekennzeichnet, daß eine Absperrfläche (40) in Form einer Kegelmantelfläche vorgesehen ist, die zur Absperrung der Kanülenbohrung (12) dient.

6. Verschlußkappe nach Anspruch 4 und 5, dadurch gekennzeichnet, daß ein O-Ring (22) von der Absperrfläche (40) bei unbetätigtem Druckknopf (26) auf die Kanülenbohrung (12) gepreßt wird.

7. Verschlußkappe nach Anspruch 6, dadurch gekennzeichnet, daß der O-Ring (22) sich an einer im Boden (6) vorgesehenen Schulter (16) abstützt.

8. Verschlußkappe nach Anspruch 1, dadurch gekennzeichnet, daß in den Absperrkörper (38) ein Trennelement (46) eingesetzt ist.

9. Verschlußkappe nach Anspruch 2, dadurch gekennzeichnet, daß im Abstand zum Boden (6) eine Zwischenwand (7, 27) im Inneren des Kappenkörpers (5) angeordnet ist, die eine Mittelbohrung (9) mit einem Ventilsitz (11) aufweist, durch welchen sich die Spitze (44) des Absperrkörpers (38) erstreckt und wobei der Ventilsitz (11) von dem Absperrkörper (38) verschließbar ist, und daß zwischen dem Boden (6) und der Zwischenwand (7, 27) ein Strömungskanal (13) gebildet ist, der die Kanülenbohrung (12) mit der Mittelbohrung (9) in der Zwischenwand (7, 27) verbindet.

10. Verschlußkappe nach Anspruch 1 oder 9, dadurch gekennzeichnet, daß auf dem Absperrkörper (38) ein Ringkragen (21) zur freigebbaren Abdichtung eines Ventilsitzes (11) vorgesehen ist.

11. Verschlußkappe nach Anspruch 8, dadurch gekennzeichnet, daß das Trennelement (46) über eine Sollbruchstelle mit dem Absperrkörper (38) verbunden ist.

## Claims

1. A cap for a tube, in particular for drawing off blood :
— having a base (6) ;
— having a cannula cone (4) arranged on the base (6) ;
— having a skirt (8) which is connected to the base (6) and which exhibits a locking device for fastening the cap (1) onto the tube (2) ;
characterized in that
— a shut-off body (38) is inserted into the skirt (8) of the cap (1) and in the non-actuated position shuts off a cannula bore (12) leading to the cannula cone (4) and clears the cannula bore (12) through the pressing down of an actuator member (26).

2. A cap as in Claim 1, characterized in that the shut-off body (38) exhibits a spike (44) which projects through the base (6) of the cap (1) and engages the actuator member (26).

3. A cap as in Claim 2, characterized in that the actuator member (26) is a pushbutton, a key, a lever or the like.

4. A cap as in Claim 3, characterized in that the spike (44) on the shut-off body (38) engages with a close fit or frictionally in a pin-receiver (36) arranged under the lid (30) of the pushbutton (26), and that the pin-receiver (36) is surrounded by a compression spring (24) bearing against the lid (30) and the base (6).

5. A cap as in one of the Claims 1 to 4, characterized in that a shut-off area (40) is provided in the form of a conical surface which serves for shutting off the cannula bore (12).

6. A cap as in Claim 4 and 5, characterized in that with the pushbutton (26) non-actuated an O-ring (22) is pressed by the shut-off area (40) against the cannula bore (12).

7. A cap as in Claim 6, characterized in that the O-ring (22) bears against a shoulder (16) provided on the base (6).

8. A cap as in Claim 1, characterized in that in the shut-off body (38) a separator member (46) is inserted.

9. A cap as in Claim 2, characterized in that a partition (7, 27) is arranged inside the body (5) of the cap at a distance from the base (6) and exhibits a drilled central hole (9) having a valve seat (11) through which the spike (44) on the shut-off body (38) extends, the valve seat (11) being able to be closed by the shut-off body (38), and that between the base (6) and the partition (7, 27) a flow channel (13) is formed, which connects the cannula bore (12) to the drilled central hole (9) in the partition (7, 27).

10. A cap as in Claim 1 or 9, characterized in that on the shut-off body (38) an annular collar (21) is provided for sealing a valve seat (11) in a clearable manner.

11. A cap as in Claim 8, characterized in that the separator member (46) is connected to the shut-off body (38) at a predetermined breaking point.

## Revendications

1. Capuchon pour tube, notamment de prélèvement sanguin, comportant :
— un fond (6),
— un cône de canule (4) disposé sur le corps (6)
— et une jupe (8) reliée au fond (6) et qui comporte un dispositif de verrouillage pour la fixation du capuchon (1) sur le tube (2), caractérisé en ce
— qu'un corps de verrouillage (38) est monté dans la jupe (8) du capuchon (1), et ce corps verrouille un alésage de canule (12) conduisant au cône de canule (4) dans sa position de repos, et libère l'alésage de canule (12) par enfoncement d'un élément d'actionnement (26).

2. Capuchon selon la revendication 1, caractérisé en ce que le corps de verrouillage (38) comporte une pointe (44), qui traverse le fond (6) du capuchon (1) et est en prise avec l'élément d'actionnement (26).

3. Capuchon selon la revendication 2, caractérisé en ce que l'élément d'actionnement (26) est un bouton-pression, un bouton-poussoir, un levier ou analogue.

4. Capuchon selon la revendication 3, caractérisé en ce que la pointe (44) du corps de verrouillage est en prise par une liaison de forme ou sous l'action d'une force avec un organe récepteur de tige (36) disposé sous le couvercle (30) du bouton pression (26), et en ce que l'organe de réception de tige (36) est entouré par un ressort de compression (24) qui prend appui entre le couvercle (30) et le fond (6).

5. Capuchon selon l'une des revendications 1 à 4, caractérisé en ce qu'il comprend une surface de verrouillage (40) en forme de cône, qui assure le verrouillage de l'alésage de canule (12).

6. Capuchon selon l'une des revendications 4 et 5, caractérisé en ce qu'une bague torique (22) est pressée sur l'alésage de canule (12) par la surface de verrouillage (40) lorsque le bouton-pression (26) est au repos.

7. Capuchon selon la revendication 6, caractérisé en ce que la bague torique (22) s'appuie sur un épaulement (16) prévu sur le fond (6).

8. Capuchon selon la revendication 1, caractérisé en ce qu'un élément de séparation (46) est disposé dans le corps de verrouillage (38).

9. Capuchon selon la revendication 2, caractérisé en ce qu'une cloison intercalaire (7, 27) est agencée à une certaine distance du fond (6) à l'intérieur du corps (5) du capuchon, et cette cloison présente un alésage central (9) muni d'un siège de soupape (11), à travers lequel s'étend la pointe (44) du corps de verrouillage (38), l'agencement étant tel que le siège de soupape (11) peut être obturé par le corps de verrouillage (38), et en ce que, entre le fond (6) et la cloison intercalaire (7, 27), il est formé un canal d'écoulement (13) qui relie l'alésage de canule (12) à l'alésage central (9) formé dans la cloison intercalaire (7, 27).

10. Capuchon selon l'une des revendications 1 et 9, caractérisé en ce qu'une collerette annulaire (21) est agencée sur le corps de verrouillage (38) pour assurer l'étanchéité du siège de soupape (11) en fonction de la position dudit corps de verrouillage.

11. Capuchon selon la revendication 8, caractérisé en ce que l'élément de séparation (46) est relié au corps de verrouillage (38) par un point destiné à la rupture.

# Fig.1

# Fig.2

Fig. 3

Fig.4